# EUROPEAN PATENT SPECIFICATION

(11) **EP 4 355 321 B1**
(45) Date of publication and mention of the grant of the patent: **09.09.2026**
(21) Application number: 22733238.4
(22) Date of filing: 16.06.2022
(51) Int. Cl.: A61K 31/405, A61K 31/4439, A61K 31/726, A61K 31/737, A61K 45/06, A61P 3/00, A61P 9/00, A61P 25/00

(54) **PHARMACEUTICAL COMPOSITION FOR INCREASING MITOCHONDRIAL ACTIVITY AND/OR IMPROVING ADIPONECTIN PRODUCTION**
PHARMAZEUTISCHE ZUSAMMENSETZUNG ZUR ERHÖHUNG DER MITOCHONDRIALEN AKTIVITÄT UND/ODER ZUR VERBESSERUNG DER ADIPONECTINPRODUKTION
COMPOSITION PHARMACEUTIQUE POUR AUGMENTER L'ACTIVITÉ MITOCHONDRIALE ET/OU AMÉLIORER LA PRODUCTION D'ADIPONECTINE

(30) Priority: 17.06.2021 NL 2028476
(43) Date of publication of application: 24.04.2024
(73) Proprietor: Logick Energetics B.V., 2333 XW Leiden (NL)
(72) Inventor: LÖWIK, Clemens Waltherus Gerardus Maria, 2333 XW Leiden (NL)
(74) Representative: Plasseraud IP
(86) International application number: PCT/NL2022/050342
(87) International publication number: WO 2022/265509

(56) References cited:
- WO-A1-2005/067943
- WO-A1-2017/105229
- WO-A2-2021/205026
- CN-B- 101 884 643
- POLITO RITA ET AL: "Adiponectin Role in Neurodegenerative Diseases: Focus on Nutrition Review", INTERNATIONAL JOURNAL OF MOLECULAR SCIENCES, vol. 21, no. 23, 4 December 2020 (2020-12-04), Basel, CH, pages 1 - 19, XP093385797, ISSN: 1422-0067, Retrieved from the Internet <URL:https://www.mdpi.com/1422-0067/21/23/9255/pdf> DOI: 10.3390/ijms21239255
- CORONA JUAN CARLOS ET AL: "PPAR[gamma] as a therapeutic target to rescue mitochondrial function in neurological disease", FREE RADICAL BIOLOGY & MEDICINE, ELSEVIER INC, US, vol. 100, 25 June 2016 (2016-06-25), pages 153 - 163, XP029789102, ISSN: 0891-5849, DOI: 10.1016/J.FREERADBIOMED.2016.06.023
- VIJAYAVEL ET AL: "Free radical scavenging activity of the marine mangrove Rhizophora apiculata bark extract with reference to naphthalene induced mitochondrial dysfunction", CHEMICO-BIOLOGICAL INTERACTIONS, ELSEVIER SCIENCE IRLAND, IR, vol. 163, no. 1-2, 27 October 2006 (2006-10-27), pages 170 - 175, XP005711156, ISSN: 0009-2797, DOI: 10.1016/J.CBI.2006.06.003
- ANONYMOUS: "Cartrophen Vet", SUMMARY OF PRODUCT CHARACTERISTICS, 21 March 2019 (2019-03-21), pages 1 - 4, XP055896544, Retrieved from the Internet <URL:chrome-extension://efaidnbmnnnibpcajpcglclefindmkaj/viewer.html?pdfurl=https%3A%2F%2Fwww.hpra.ie%2Fimg%2Fuploaded%2Fswedocuments%2FLicence_VPA22748-001-001_20032019162108.pdf&clen=77995&chunk=true> [retrieved on 20220301]
- ZARGHAMRAVANBAKHSH PARIA ET AL: "Metabolic causes and consequences of nonalcoholic fatty liver disease (NAFLD)", METABOLISM OPEN, vol. 12, 1 December 2021 (2021-12-01), pages 1 - 7, XP055957360, ISSN: 2589-9368, Retrieved from the Internet <URL:https://www.sciencedirect.com/science/article/pii/S2589936821000736#:~:text=Abstract,2%20diabetes%20mellitus%20(T2DM).> DOI: 10.1016/j.metop.2021.100149

## Description

### FIELD OF THE INVENTION

The invention relates to a pharmaceutical composition for increasing mitochondrial activity and/or improving adiponectin production. It has been found that the present composition contributes positively in the recovery, the treatment, and reducing negative effects of such cell-related diseases and disorders, in particular in a neurodegenerative disorder.

### BACKGROUND OF THE INVENTION

A mitochondrion is a double membrane-bound organelle found in cells of most eukaryotic organisms. Mitochondria are considered to generate the cell's energy levels, e.g. by supply of adenosine triphosphate (ATP). ATP is then used as a source of energy by conversion to adenosine diphosphate (ADP). In addition to supplying cellular energy, mitochondria may be involved in further tasks, such as signalling, cellular differentiation, cell death, maintaining control of the cell cycle, and controlling cell growth. The mitochondrion on its turn typically has compartments for carrying out even further specialized functions. These compartments or regions may relate to the outer membrane, the intermembrane space, the inner membrane, etc.

It is noted that not all cells, and likewise, not all unicellular organisms, contain mitochondria, such as some red blood cells, or some specific organisms.

Mitochondria are relatively small cell constituents, typically between 0.75 and 3 µm in area, with possible variable size and structure difference between mitochondria. Also, a number of mitochondria in a cell can vary widely, by organism, by tissue, and by cell type, from 0 up to even 2000.

Despite their size, they are difficult to observe, even under a microscope or the like. Mitochondrial biogenesis is in turn temporally coordinated with these cellular processes.

Sub-optimal functioning of mitochondria has been associated with certain human diseases and conditions, such as cardiac dysfunction, heart failure and neurodegenerative diseases.

Like a cell, with its nuclear DNA, a mitochondrion has a genome. This genome is rather similar to a bacterial genome. Likewise, a mitochondrion can transcribe proteins and the like from mitochondrial DNA. As mentioned in view of mitochondrial variation, also transcription may vary widely, e.g. depending on tissue comprising said mitochondrion or a species comprising said mitochondrion. In humans, many distinct types of proteins have been identified. The mitochondrial proteome is considered to be dynamically regulated.

Adiponectin (also referred to as GBP-28, apM1, AdipoQ and Acrp30) is a protein hormone and adipokine, which is involved in regulating glucose levels as well as fatty acid breakdown. In humans it is encoded by the ADIPOQ gene and it is produced primarily in adipose tissue, but also in muscle, and even in the brain.

Adiponectin is a polypeptide. Adiponectin typically forms larger structures, such as by forming trimers, hexamers or even dodecamers. In the adiponectin molecule four distinct regions can be recognized. A first is considered to target hormone for secretion outside a cell, a second is considered to vary between species, a third is considered similar to collagenous proteins, and a fourth is considered to be a globular domain. So, a part of the adiponectin function is modulation of a number of metabolic processes by secretion of said hormone. An example of such a metabolic process is glucose regulation and fatty acid oxidation. Adiponectin is secreted from adipose tissue typically into the bloodstream. Therefore, its presence is abundant in plasma, relative to e.g., other hormones. Its relative levels are about 0.01% of all plasma proteins, and its absolute levels are about 5-10 µg/mL. In adults, plasma concentrations are found to be higher in females than males, and they are reduced in diabetics compared to non-diabetics. Weight reduction is found to significantly increases circulating concentrations. Although studies have found adiponectin to be inversely correlated with body mass index in patient populations, this was not confirmed in healthy adults. Circulating adiponectin concentrations, e.g., in the plasma, are found to increase during limited caloric uptake/intake in animals and humans.

Some mice models, specifically for transgenic mice models, having an increased adiponectin level, show amongst others a reduced adipocyte differentiation, and an increased energy expenditure, which latter is considered to be associated with mitochondrial uncoupling. It is considered that the adiponectin hormone plays a role in many disorders and diseases, such as in the suppression of metabolic derangements, atherosclerosis, non-alcoholic fatty liver disease (NAFLD), as an independent risk factor for metabolic syndrome, in insulin resistance in mice in fatty acid oxidation, and in suppression of hepatic glucose production. High-molecular weight forms of adiponectin may be associated to glucose homeostasis, may be associated with a lower risk of diabetes with similar magnitude of association as total adiponectin. Coronary artery disease has been found to be positively associated with high molecular weight adiponectin, but not with low molecular weight adiponectin. So, in some aspects adiponectin has positive effects, and in some aspects negative effects.

Some documents may be relied on. WO 2017/105229 A1, of the present inventors, recites a pharmaceutical composition, comprising (i) a compound comprising at least one carbohydrate and containing at least one sulphate, in combination with (ii) at least one non-protein or non-NA-strand compound, different from (i), that can activate PPAR, preferably PPARγ, and (iii) optionally, at least one pharmaceutically acceptable carrier. Preferably, the sulphated compounds include single sulphur containing agents like MSM or multiple sulphur containing agents like pentosan, adequan. The second part of the composition, is a PPARy agonists chosen from the group of thiazolidinediones, NSAIDs, sulphonylureas and indoles, like for example indomethacin or pioglitazone. CN 101 884 643 B recites the application of pioglitazone and heparin or low molecular heparin or pharmaceutically acceptable salts thereof or derivatives thereof to preparing a pharmaceutical composition, in particular to the application to preparing a medicament for preventing or treating fatty liver. the purpose of the pioglitazone and the heparin or the low molecular heparin or the pharmaceutically acceptable salts thereof in lessening fat deposition in rat hepatocytes and the synergy of a pioglitazone and heparin or low molecular heparin compound in preventing or treating the rat fatty liver are proved by animal tests. WO 2005/067943 A1 recites pharmaceutical compositions comprising of glycosaminoglycan or salts thereof, preferably chondroitin or salts thereof, more preferably chondroitin sulphate, and nonsteroidal anti-inflammatory drug(s) or salts thereof, optionally with pharmaceutically acceptable excipient(s) are described. The paper by Corona JC and Duchen MR (Free Radical Biology and Medicine 2016 (100):153-163) reviews PPARγ as a therapeutic target for rescuing mitochondrial function in neurological disease. It summarises evidence that PPARγ agonists may provide neuroprotection in neurodegenerative disorders through effects including modulation of mitochondrial dysfunction and oxidative stress.

As treatment for increasing mitochondrial activity or improving adiponectin production is scarce or ineffective, there still is a need for improved pharmaceuticals and treatments which overcome drawbacks of the prior art.

### SUMMARY OF THE INVENTION

It is an objective of the invention to provide a pharmaceutical composition, as well as a dosage comprising said pharmaceutical composition, and a medicament for increasing mitochondrial activity, such as oxygen consumption rate, mitochondrial fatty acid synthesis, and ketoreductase, or improving adiponectin production, and said pharmaceutical composition as a medicament. The invention is set out in the appended set of claims.
In particular, the invention describes a pharmaceutical composition, comprising (i) pentosan polysulphate (CAS 37300-21-3 N or 116001-96-8, (C₅H₆Na₂O₁₀S₂)ₙ, n=1-10), in combination with (ii) a second compound selected from indomethacin (CAS 53-86-1) and pioglitazone (CAS 112529-15-4), wherein the pentosan polysulphate and the second compound are provided in a molar ratio of 0.01:1 to 1:0.01, preferably in a weight ratio of 1:0.1 to 0.9:1, more preferably in a weight ratio of 1:0.3 to 1:1, such as 1:0.5 to 1:0.7. Inventors discovered that virus induced infections and disorders can be treated by a composition according to the invention. It is noted that various documents refer to pentosan polysulphates and second compounds according to the invention individually, but not in combination. In fact, a combination of the two is typically discouraged. The synergistic effect of the present invention could therefore not be observed. The synergistic effect is for instance found in a lower combined dosage, compared to the sum of the two individual dosages, that is required to find the same effect, typically a 2-10 times lower dosage, which lower dosage e.g., mitigates toxic side effects, the increased activity of the at least one second compound induced by the at least one first compound, and the decrease of side effects per se, in particular of the at least second compound. The effect of the present pharmaceutical composition may e.g. be evidenced by a Protein Coding gene, such as BNIP's, such as BNIP3 (BCL2 Interacting Protein 3, wherein BL2 is B-cell lymphoma 2, encoded in humans by the BCL2 gene, is a member of the Bcl-2 family of regulator proteins that regulate cell death), by a fibroblast growth factor protein, such as FGF21, or G protein-coupled receptors, such as GPR120, or Sirtuin 1 Proteins, e.g. SIRT1 protein, or ATP levels, or GDF15 levels.

Hence, patients with disturbed mitochondrial activity or disturbed adiponectin production, benefit from use of the pharmaceutical composition of the invention.

Therefore, the invention relates to the pharmaceutical composition of the invention for use in the treatment of a mitochondrial activity or adiponectin production related disorder or disease, wherein said disease or disorder is a neurodegenerative disease.

In a further aspect the present disclosure relates to a dosage comprising separate dosage forms for individual pharmaceutical active ingredients, and/or wherein the composition is in the form of a tablet, capsule, repository, nanoparticles, or injectable.

### DETAILED DESCRIPTION OF THE INVENTION

In the present pharmaceutical composition for use in the treatment of a mitochondrial activity related disease or disorder and/or of an adiponectin production related disease or disorder, the first compound is pentosan polysulphate (CAS 37300-21-3 N or 116001-96-8, (C₅H₆Na₂O₁₀S₂)ₙ, n=1-10).

In an exemplary embodiment of the present pharmaceutical composition for use in the treatment of a mitochondrial activity related disease or disorder and/or of an adiponectin production related disease or disorder the at least one second compound can activate PPARy.

In the present pharmaceutical composition for use in the treatment of a mitochondrial activity related disease or disorder and/or of an adiponectin production related disease or disorder the second compound is selected from indomethacin (CAS 53-86-1), pioglitazone (CAS 112529-15-4), and combinations thereof.

In the present pharmaceutical composition for use in the treatment of a mitochondrial activity related disease or disorder and/or of an adiponectin production related disease or disorder the first compound and second compound is provided in a molar ratio of 0.01:1 to 1:0.01.

In an exemplary embodiment the present pharmaceutical composition for use in the treatment of a mitochondrial activity related disease or disorder and/or of an adiponectin production related disease or disorder the composition further comprises (iii) at least one pharmaceutically acceptable carrier.

In an exemplary embodiment of the present pharmaceutical composition for use in the treatment of a mitochondrial activity related disease or disorder and/or of an adiponectin production related disease or disorder the active pharmaceutical ingredients may be in one dosage form, preferably comprising 1-10 mg active ingredients/kg body weight, such as 2-1000 mg active ingredients.

In the present pharmaceutical composition for use in the treatment of a mitochondrial activity related disease or disorder and/or of an adiponectin production related disease or disorder comprises pentosan polysulphate, and indomethacin and/or pioglitazone.

In an exemplary embodiment the present pharmaceutical composition for use in the treatment of a mitochondrial activity related disease or disorder and/or of an adiponectin production related disease or disorder the pharmaceutical composition may comprise separate dosage forms for individual pharmaceutical active ingredients.

In an exemplary embodiment of the present pharmaceutical composition for use in the treatment of a mitochondrial activity related disease or disorder and/or of an adiponectin production related disease or disorder the dosage may be in the form of a tablet, capsule, repository, nanoparticles, or injectable.

In an exemplary embodiment of the present pharmaceutical composition for use in the treatment of a mitochondrial activity related disease or disorder and/or of an adiponectin production related disease or disorder the composition may be in the form of a tablet or capsule suitable for oral administration.

In an exemplary embodiment of the present pharmaceutical composition for use in the treatment of a mitochondrial activity related disease or disorder and/or of an adiponectin production related disease or disorder may be for use as a medicament by administering said medicament in an effective amount for a sufficient period.

In an exemplary embodiment of the present pharmaceutical composition for use in the treatment of a mitochondrial activity related disease or disorder and/or of an adiponectin production related disease or disorder the administration may be to a pet or mammal.

The present pharmaceutical composition is for use in the treatment of a mitochondrial activity related disease or disorder and/or of an adiponectin production related disease or disorder, wherein the disease or disorder is a neurodegenerative disease. The disorder or disease may be in chronic form, in acute form, in a transitional form, such as from one disorder to another, or in any other form.

The pharmaceutical composition according the invention may be a combination composition, wherein the active pharmaceutical ingredients are in one dosage form. The pharmaceutical composition may also comprise separate dosage forms for individual pharmaceutical active ingredients.

In an exemplary embodiment of the present pharmaceutical composition the active pharmaceutical ingredients may be in one dosage form, preferably comprising 0.001-10 mg active ingredients/kg body weight, preferably 0.01-5 mg active ingredients/kg body weight, more preferably 0.05-2 mg active ingredients/kg body weight, even more preferably 0.1-1 mg active ingredients/kg body weight, such as 0.2- 0.5 mg/kg, such as 0.01-100 mg active ingredients.

In an exemplary embodiment of the present dosage the dosage is for sub-cutaneous application, such as a weekly application, wherein a sub-cutaneous dosage comprises 20-100 mg active ingredient per dosage.

In an exemplary embodiment of the present dosage the at least one first compound and at least one second compound are provided in a weight ratio of 1:1 to 10:1, preferably in a weight ratio of 1.5:1 to 5:1, and wherein a total weight of active ingredients is from 1-100 mg per dosage.

The compound that activates PPAR, preferably PPARγ preferably is a compound that can activate PPAR, meaning the PPAR receptor activity increases by at least 2-3 times compared to a baseline situation without the PPAR activating compound.

The compound that activates PPAR, preferably PPARγ preferably has anti-inflammatory effects, meaning that at least one of the inflammatory markers is reduced. Inflammatory markers are e.g. TNFα, IFN, cytokines, histamine, interleukins, chemokines, leukotrienes, lysosome granules and prostaglandins.

A carbohydrate is an organic compound comprising only carbon, hydrogen, and oxygen, usually with a hydrogen:oxygen atom ratio of 2:1 (as in water); with the empirical formula C*ₘ*(H₂O)*ₙ* (where *m* could be different from *n*). Structurally it is more accurate to view carbohydrates as polyhydroxy aldehydes and ketones.

Carbohydrates as used in the present invention do not relate to glycosylated proteins or nucleoside compounds (like DNA, RNA or the like)

The carbohydrate is a Glycosaminoglycans (GAGs). Glycosaminoglycans, or mucopolysaccharides are long unbranched polysaccharides consisting of a repeating disaccharide unit. The repeating unit consists of an amino sugar (N-acetylglucosamine or N-acetyl galactosamine) along with a uronic sugar (glucuronic acid or iduronic acid) or galactose.

Based on core disaccharide structures, GAGs may be classified into four groups.

Preferred types of sulphated saccharides are also known as heparin, heparan sulphate analogues, or heparin-like compounds.

Suitable compounds comprising a carbohydrate and a sulphate group can be derived from natural sources, or can be made - at least in part - synthetically.

Examples of sulphated compounds include single Sulphur containing agents like MSM (dimethylsulfon), dextran sulphate, or multiple Sulphur containing agents, like polysulphated glycosaminoglycan (adequan sulphate), heparin like pentosan sulphate or the like. The carbohydrate or first compound according to the present invention is pentosan polysulphate.

The second part of the present composition, is an agent that can activate PPAR, preferably PPARγ. The PPAR agonists of the present invention are selected from pioglitazone and indomethacin.

Preferably, both the first compound and the second compound that activates PPARγ have a combined, synergistic effect.

Whether a compound is a PPAR, preferably PPARγ agonist can be determined in a simple cell-based test.

By PPAR agonist is meant any compound that increases the biological activity or expression of one or more PPARs (e. g., PPARα, PPARy, and PPARβ/δ) in a cell by a least 10% relative.

Examples of PPARy agonists include any of the Thiazolidinediones, but particularly Rosiglitazone, Troglitazone, and Pioglitazone and analogs thereof. Additional examples of PPARγ agonists include non-steroidal anti-inflammatory drugs, such as Indomethacin, Ibuprofen, Naprosyn and Fenoprofen and antioxidants such as vitamin E, vitamin C, S-adenosyl methionine, selenium, beta-carotene, idebenone, cysteine, dithioerythritol, dithionite, dithiothreitol, and pyrosulfite.

Examples of PPARα agonists include any of the fibrates (e.g., fenofibrate, bezafibrate, gemfibrozil, and analogs thereof), docosahexaenoic acid, and Wy 4643.

So, for example Pentosan polysulphate in combination with one of these compounds could be used in the treatment disclosed herein. More specifically PPARγ agonistic action can be determined in a similar manner as described above, using a dual luciferase assay with a luciferase construct containing a PPARγ responsive element.

This second compound is not a protein or nucleic acid-based compound.

Suitable PPARγ agonists include thiazolidinediones, NSAIDs, sulphonyl ureas and indoles.

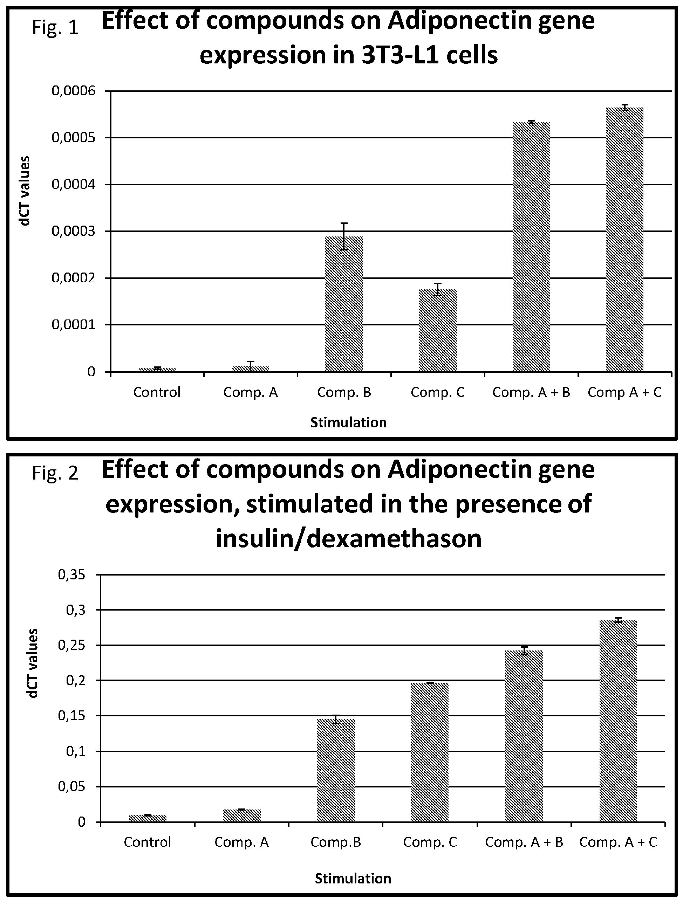

Thiazolidinediones (abbreviated as TZDs) are also known as glitazones. Suitable thiazolidinediones include rosiglitazone, pioglitazone, troglitazone, and ciglitazone, which are selective ligands for the nuclear transcription factor peroxisome proliferator- activated receptor (PPAR)y. Typical TZDs have the formula as shown in fig. 1, where n is 1,2, or 3, Y and Z independently are O or NH; and E is a cyclic or bicyclic aromatic or non-aromatic ring, optionally containing a heteroatom selected from oxygen or nitrogen. Suitable TZDs are for example described in WO2000/27401.

NSAIDs are a class of drugs that provides analgesic and antipyretic (fever-reducing) effects, and, in higher doses, anti-inflammatory effects. PPARγ is activated by several endogenous ligands emerging from the metabolism of arachidonic acid and linoleic acid. Among the PPARγ ligands represented are the lipoxygenase products 13(S)HODE (produced from linoleic acid by 15-LOX-1) and 15(S)HETE (produced from arachidonic acid by both 15-LOX-1 and 15-LOX-2, although 15-LOX-2 catalyzes this reaction much more efficiently). Induction of 15-LOX-1 activity by NSAIDs occurs independently of COX-2 inhibition

NSAIDs can be classified based on their chemical structure or mechanism of action. Older NSAIDs were known long before their mechanism of action was elucidated and were for this reason classified by chemical structure or origin. Newer substances are more often classified by mechanism of action.

Suitable NSAIDs include salicylates, propionic acid derivatives, acetic acid derivatives, enolic acid (oxicam) derivatives and fenamic acid derivatives,

Suitable salicylates include aspirin (acetylsalicylic acid), diflunisal (Dolobid^{™}), salsalate (Disalcid^{™}) and choline magnesium trisalicylate (Trilisate^{™})

Suitable propionic acid derivatives include ibuprofen, dexibuprofen, naproxen, fenoprofen, ketoprofen, dexketoprofen, flurbiprofen, oxaprozin, and loxoprofen.

Suitable acetic acid derivatives include Indomethacin, tolmetin, sulindac, etodolac, ketorolac, diclofenac and nabumetone (drug itself is non-acidic but the active, principal metabolite has a carboxylic acid group)

Suitable enolic acid (oxicam) derivatives include piroxicam, meloxicam, tenoxicam, droxicam, lornoxicam, and isoxicam.

Suitable fenamic acid derivatives (fenamates ) include mefenamic acid, meclofenamic acid, flufenamic acid, and tolfenamic acid.

Sulfonylurea derivatives are a class of antidiabetic drugs that are used in the management of diabetes mellitus type 2. Examples include carbutamide, acetohexamide, chlorpropamide, tolbutamide, tolazamide, glipizide, gliclazide, glibenclamide (glyburide), glibornuride, gliquidone, glisoxepide, glyclopyramide and glimepiride. They act primarily by increasing insulin release from the beta cells in the pancreas. All sulfonylureas contain a central S-phenyl sulfonylurea structure with a p- substituent on the phenyl ring (R) and various groups terminating the urea N' end group (R2) (see figure 2).

Indoles include indole-derived agents which can bind to PPARγ, and comprise mainly sulfonyl-indoles.

Dual alpha-gamma agonists are suitable as well, and include glitazars.

Suitable glitazars include aleglitazar, muraglitazar and tesaglitazar.

PPARα agonists include fibrates and biguadines.

Preferably, the compound comprising at least one saccharide and at least one sulphate can be present in the pharmaceutical composition such as in an amount of 0-10 wt.% relative to a total weight.

Preferably, the PPARγ agonist can be present in an amount of 0-10 wt.% relative to a total weight.

Preferably, the relative amounts between the two compounds (sulphated compound to PPARγ agonist) are 1:1 to 1:5.

The pharmaceutically acceptable carrier can be present in an amount of 0.01% to 99.9%, preferably 0.1%-10%, and its amount will depend on the formulation.

In one embodiment, the medicament is in the form of a tablet, suitable for oral administration.

In another embodiment, the medicament is in a form suitable for local administration.

The medicament can comprise the components in the form of a solid, or liquid preparation.

The dosage form can be an immediate release or extended-release formulation.

It is an advantage of the present invention that the constituents of the composition can be non-biologics, and several of the components are approved medicaments.

Pentosan polysulphate (PPS), manufactured from beech-wood, is an FDA-approved oral medication for the treatment of interstitial cystitis (IC), also known as painful bladder syndrome. PPS is known to have anti-inflammatory and pro-chondrogenic properties. Pentosan polysulphate is available as pills or as a direct infusion into the bladder.

Indomethacin is an FDA-approved, non-steroidal anti-inflammatory drug (NSAID). It's commonly used to reduce fever, pain, stiffness and swelling. Furthermore, Indomethacin is a COX-inhibitor that blocks prostaglandin production and is used as an inhibitor of inflammation. In higher doses it can also induce adipogenesis in vitro in mesenchymal progenitor cells by activating PPARγ.

Pioglitazone is a powerful PPARγ agonist, and belongs to the class of thiazolidinediones, or glitazones, and was designed to treat type II diabetes by increasing the insulin sensitivity.

### SUMMARY OF FIGURES

Figures 1-4 show experimental results.

### DETAILED DESCRIPTION OF THE FIGURES

### qPCR results in KS-483 cells and 3T3-L1 cells

### Experimental design cell culture for RNA isolation 3T3-L1 cells

### Protocol 3T3-L1 cell line

DMEM
10 % heat inactivated FCS
Compound A - 5,0 µg/ml
Compound B - 50 µM
Compound C - 1,0 µM
Compound A + B
Compound A + C

### Protocol 3T3-L1 cell line

DMEM
10 % heat inactivated FCS
Compound A - 0,5 µg/ml
Compound B - 50 µM
Compound C - 1,0 µM
Compound A + B
Compound A + C

In the of presence of:
0.25 µM Dexamethasone &
1 ug/ml Insulin
   Therein A=pentosan, B=indomethacin, and C= pioglitazone.

Cells were stimulated at day 4 when they were sub confluent. After 72 hours of incubation, RNA was isolated from the cells.

Fig. 1 shows the effect of compounds on Adiponectin gene expression in 3T3-L1 cells. Compound A and the control show hardly any effect. Compounds B and C show adiponectin gene expression, whereas the present combination A+B and A+C, respectively, show a clear synergistic effect on the adiponectin gene expression.

Fig. 2 is in line with fig. 1. Therein the effect of compounds on Adiponectin gene expression, stimulated in the presence of insulin/dexamethasone. The results are comparable with that of fig. 1.

### Experiment set up KS-483 cells:

Medium: αMEM supplemented with 10 % heat inactivated FCS

Cells were stimulated on day 4 when they reached confluency. At day 7, cells were harvested for RNA isolation.

Concentration compounds in KS-483 cells for RNA isolation:
Compound A 5 µg/ml
Compound B 10 uM
Compound C 1 uM.

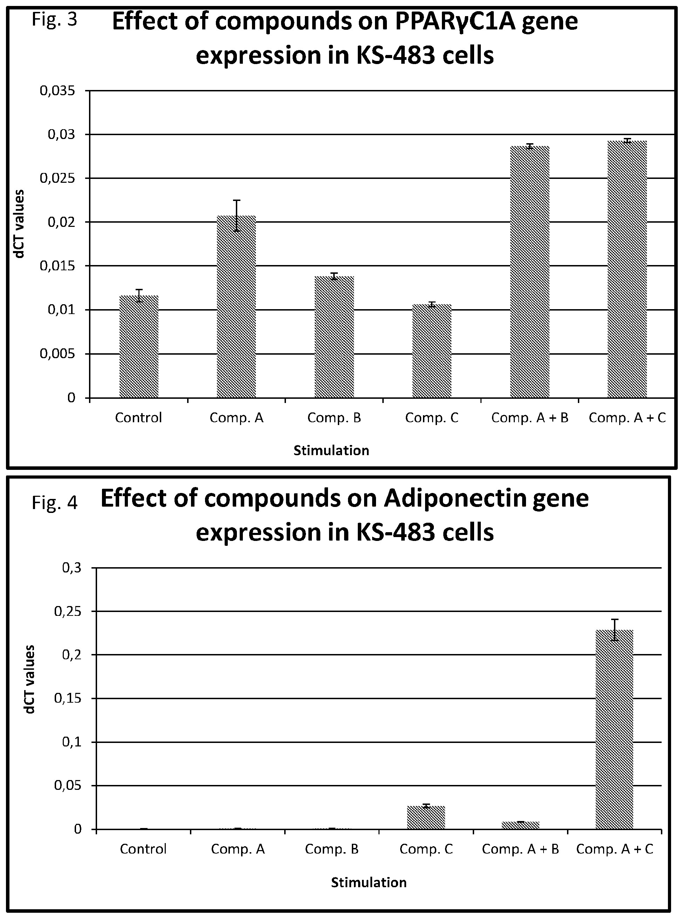

Fig. 3 shows the effect of compounds on PPARγC1A gene expression in KS-483 cells. Compounds A-C and the control each individually show some effect, in the range of the control, whereas the combined compounds A+B and A+C, respectively show an increased expression.

In fig. 4 the effect is much more dramatic. The control, and compounds A-B each individually show no measurable effect, compound C shows some effect, whereas compounds A+B do show an effect and compounds A+C show a large increase.

In summary, the present pharmaceutical composition show a synergistic effect on the adiponectin production, evidenced by the gene expression.

### Experimental set-up

The present first and second compound, such as pentosan, is typically provided in the drinking water, such as at a dosage of 20-25 mg/kg/day, and/or sub-cutaneous of e.g. 50 mg/kg/week.

### Experiment:

Day 1 baseline blood draws and intra tracheal injection of 80 µl of physiological saline (control for bleomycin) with Isoflurane anesthesia.
Day 7 baseline blood draws
Day 7 start treating with compounds (n=28, n=4 per group)
   1 compound B (second compound, e.g. indomethacin) 4mg/kg (DMSO and trapsol) drinking water
   2 compound C (alternative/additional second compound) 40mg/kg (DMSO and trapsol) drinking water
   3 compound A (first compound) 20mg/kg and B 4mg/kg (DMSO and trapsol) drinking water
   4 compound A 20mg/kg and C 40mg/kg (DMSO and trapsol) drinking water
   5 compound A 50mg/kg/week subcutaneously weekly and B 4mg/kg (DMSO and trapsol) drinking water
   6 compound A 50mg/kg/week subcutaneously weekly and C 40mg/kg (DMSO and trapsol) drinking water
   7 control (DMSO and trapsol) saline subcutaneous weekly + drinking water (added because inventors need a control group without compounds to compare with given FACS BAL dif staining and activation markers on the alveolar macrophages)

Day 14 change drinking water and subcutaneous injections with A

### Day 21 sacrifice mice.

Collection of:
- Blood divided into 3 portions (Acrp30 (5µl), cytokines (50µl) and remainder); measure at regular intervals, typically 5 times, whereof 2 times after treatment starts; measure e.g., adiponectin levels. Adipocyte complement-related protein of 30 kDa (Acrp30, adiponectin, or AdipoQ) is a fat-derived secreted protein that circulates in plasma. Acrp30 is lower in insulin-resistant states and it is implicated in the regulation of in vivo insulin sensitivity. Plasma Acrp30 levels from two diabetic mouse models were increased in response to treatment.

Results suggest that induction of Acrp30 may represent a key mechanism that contributes to the beneficial metabolic effects of the present compounds and that measurement of Acrp30 levels prove to be a valuable biomarker that can be used to gauge the extent of in vivo activation.
- FACS analysis (fluorescence activated cell sorting) of cells in BAL (bronchoalveolar lavage), a ELISA (enzyme-linked immunosorbent assay) in bronchoalveolar fluid (BALF).
- Lung histology (freeze) and left lobe for hydroxyproline assay.

### Results

From the experiments it follows that first of all a positive effect was observed in the mitochondrial activity and likewise the adiponectin levels. In addition, the combined pharmaceutical composition of the present invention showed a synergistic effect over the individually applied first and second compounds, respectively. The synergistic effect of the present composition was typically at least 2 times the summed individual effects, mostly at least 10 times, and often at least 20 times, hence at least a factor higher.

### Measurement of mitochondrial activity

For measuring the mitochondrial activity use is made of a so-called Seahorse XF Cell Mito Stress Test Kit of Agilent Ltd. KS483 mesenchymal progenitor cells were measured on their activity. A comparison is made between KS483 cells as such (no treatment, blank), KS483 cells with either of the present compounds, e.g. with pentosan polysulphate, or adequan polysulphate, and indomethacin, or pioglitazone, respectively, and with both of the present compounds. Typically undifferentiated KS4583 cells and adipocyte differentiated KS 483 cells were used in experimental set-ups. Apart from KS483 cells HT22 cells, a murine hippocampal cell line that is a subclone of HT4 cells was used.

### Protocol seahorse experiments:

Seahorse XF analyzer simultaneously monitors the 2 major energy pathways in the cell, that is mitochondrial respiration and glycolysis. This is done by measuring the rate of oxygen consumption and proton release into the medium that surrounds the cell or extracellular flux. By measuring the extracellular flux of analytes, oxygen and protons you can determine the bioenergetic status of the cell without adding labels that can alter cell behavior.

Mitochondrial respiration or oxygen metabolism is one of the key ways a cell generates energy. Where oxygen is present the cells respire to generate ATP. Consuming oxygen and energy substrates like free fatty acids, glucose and glutamine. The analyzer provides a kinetic measurement of respiration measuring the rate glucose is consumed from the medium, what is called the Oxygen Consumption Rate (OCR). Cells also generate ATP through glycolysis. The conversion of glucose into lactate independent of oxygen. Lactate is the primary source of free protons in the medium of cultured cells. The analyzer also kinetically measures lactate acid production by measuring the rate free protons are released which acidifies the medium. Inventors call this the Extra Cellular Acidification Rate (ECAR).

The analyzer thus can measure the metabolic phenotype of cells by simultaneously measuring respiration and glycolysis in real-time and the shift between the 2 pathways under pathological states enables you to connect physiological trades of cells, with genomic and proteomic data to create new insights into diabetes, obesity, cancer, cardiovascular and neurodegenerative function and safety toxicity.

### Cell culture.

HT-22 cells were maintained cells in Dulbecco's modified Eagle medium (HyClone, Logan, UT) supplemented with 10% fetal bovine serum (HyClone) and penicillin-streptomycin solution in monolayers in 10-cm Greiner tissue culture dishes (Orlando, FL) under standard cell culture conditions (5% CO2, 95% air). Inventors changed the medium three times weekly and back-cultured at confluence (every 3-5 days). Inventors observed cells with a phase-contrast microscope (Zeiss Observer Z1). Inventors used HT-22 cells at passages 10-30.

### Cellular bioenergetics (Seahorse).

### Oxygen Consumption Rate.

Inventors plated HT-22 cells at a density of 30,000/well on an XF24 plate. Cells attached overnight, and inventors exchanged the media 1 h before the assay for XF24 media. Inventors diluted rotenone (final concentration, 0.1 µM), FCCP (final concentration, 0.3 µM), and oligomycin (final concentration, 1 µg/ml) into XF24 media and loaded into the accompanying cartridge. Injection of drugs into the medium occurred at the time points specified. Inventors monitored oxygen consumption (OCR) with a Seahorse Bioscience XF24 Extracellular Flux Analyzer. Inventors compared the vehicle- and drug and drug combinations-treated groups with the paired t test.

### Glycolysis.

Glycolysis of treated (drug and drug combinations) and untreated cells was measured with a XF-96 extracellular flux analyzer SeaHorse. According to manufacturer's recommended protocol of Seahorse XF96 extracellular flux analyzer, cell medium was replaced by the conditional medium and incubated at the incubator without supplied CO2 for one hour before completion of probe cartridge calibration. Extracellular acidification rate (ECAR) was measured in the Seahorse XF96 Flux analyzer. Measurements were performed after injection of three compounds affecting bioenergetics: glucose, oligomycin and 2DG (Sigma, St Louis, MO, USA) and the measurements were normalized against the cell densities. Each experiment contained triplicate data points.

### Glucose uptake.

Inventors determined the effect of our drugs and drug combinations on glucose uptake by using the 2-NBDG assay. Inventors plated HT-22 cells onto 25-mm coverslips at a density of 2.5×104 cells/coverslip and allowed them to attach overnight. The next day, inventors incubated cells in glucose-free Krebs Ringer HEPES (KRH) buffer (129 mM NaCl, 5 mM NaHCO3, 4.8 mM KCl, 1.2 mM KH2PO4, 1 mM CaCl2, 1.2 mM MgCl2, 10 mM HEPES) for 30 min. Afterward, inventors incubated cells for 5 min at 37°C in KRH buffer containing 100 µM 2-NBDG and specific concentrations of the drugs and drug combinations. Inventors washed the cells three times in KRH buffer and mounted them on a coverslip. Inventors took images with a Zeiss Observer Z1 microscope. Inventors used ImageJ software to quantify the 2-NBDG by dividing the cellular fluorescence by the total cell number. Inventors compared the vehicle- and MB-treated groups with the paired t test.

### Measurement of adiponectin levels

For measuring the adiponectin levels use is made of an Arcp30 Elisa. Cells were measured on their activity. A comparison is made between cells as such (no treatment, blank), cells with either of the present compounds, e.g. with pentosan polysulphate, or adequan polysulphate, and indomethacin, or pioglitazone, respectively, and with both of the present compounds. The measurements can be performed as mentioned above.

### Effect on Mitochondrial activity

The below table shows the synergistic effect of the combined present pharmaceutical composition. Therein A=pentosan, B=indomethacin, and C= pioglitazone. What is shown in the table if log2Foldchanges. The RNA is isolated after 3 and 7 days stimulation in KS-cells, respectively. Ppargcla is considered particularly relevant for mitochondrial activity. A higher value represents a higher activity.

| Ppargc1a | A | B | C | A+B | A+C |
|---|---|---|---|---|---|
| 3days | 1.14 | - | - | 1.84 | 1.71 |
| 7days | 1.3 | - | - | 2.19 | 2.17 |

From the table it is clear that component A alone has some effect of the changes in Ppargscla activity, whereas components B and C have no measurable effect. Surprisingly, A+B, as well as A+C have a significant increased effect. This is considered clear evidence of the synergistic effect of the combined present pharmaceutical composition of the mitochondrial activity. It is noted that the mitochondria's purpose is to produce energy.

For further gene expression Log2fold change data show the following data (after 7 days of stimulation of KS483 cells, for the Nduf and Cox families of sub-unit proteins of mitochondrial complex 1 and complex 4, respectively:

| gene | A | B | C | A+B | A+C |
|---|---|---|---|---|---|
| COX5a | 0.31 | 0 | 0 | 0.43 | 0.42 |
| COX15 | 0 | 0 | 0 | 0.5 | 0.54 |
| COX6b 1 | 0 | 0 | 0 | 0.44 | 0.4 |
| COX7a2L | -0.42 | 0 | 0 | 0 | -0.52 |
| COX5b | 0 | 0 | 0 | 0.58 | 0 |
| COX20 | 0 | 0 | 0 | 0.58 | 0.59 |
| COX17 | 0 | 0 | 0 | 0.68 | 0.68 |
| COX8a | 0 | 0 | 0 | 0.54 | 0.61 |
| COX4iL | 0 | 0 | 0 | 0.28 | 0 |
| COX19 | 0 | 0 | 0 | 0.47 | 0 |
| COX8b | | | | | |

| gene | A | B | C | A+B | A+C |
|---|---|---|---|---|---|
| Ndufb10 | 0 | 0 | 0 | | 0.28 |
| Ndufaf7 | 0 | 0 | 0.35 | | 0.34 |
| Ndufs10 | 0 | 0 | 0 | | 0.52 |
| Ndufa10 | 0 | 0 | 0 | | 0.54 |
| Ndufs20 | 0 | 0 | 0 | | 0.3 |
| Ndufb4 | 0 | 0 | 0 | | 0.41 |
| Ndufb3 | 0 | 0 | 0 | | 0.45 |
| Nduf4 0 | 0 | 0 | 0 | | 0.4 |
| Ndufc1 | 0 | 0 | 0 | | 0.44 |
| Ndufab1 | 0 | 0 | 0 | | 0.53 |
| Ndufv3 | 0 | 0 | 0 | | 0.71 |
| Ndufv1 | 0 | 0 | 0 | | 0.38 |
| Ndufa9 | 0 | 0 | 0 | | 0.35 |
| Ndufb9 | 0 | 0 | 0 | 0.49 | 0.4 |
| Ndufa5 | 0 | 0 | 0.57 | 0 | 0 |

The results are considered to be rather remarkable, and in line with the above results.

## Claims

1. A pharmaceutical composition for use in the treatment of a mitochondrial activity related disease or disorder and/or of an adiponectin production related disease or disorder, comprising
(i) pentosan polysulphate (CAS 37300-21-3 N or 116001-96-8, (C₅H₆Na₂O₁₀S₂)ₙ, n=1-10), in combination with
(ii) a second compound selected from indomethacin (CAS 53-86-1) and pioglitazone (CAS 112529-15-4),
wherein the pentosan polysulphate and the second compound are provided in a molar ratio of 0.01:1 to 1:0.01, preferably in a weight ratio of 1:0.1 to 0.9:1, more preferably in a weight ratio of 1:0.3 to 1:1, such as 1:0.5 to 1:0.7, wherein the disease or disorder is a neurodegenerative disease.

2. The pharmaceutical composition for use in the treatment of a mitochondrial activity related disease or disorder and/or of an adiponectin production related disease or disorder according to claim 1, wherein the composition further comprises (iii) at least one pharmaceutically acceptable carrier.

## Patentansprüche

1. Pharmazeutische Zusammensetzung zur Verwendung bei der Behandlung einer mit mitochondrialer Aktivität in Zusammenhang stehenden Krankheit oder Störung und/oder einer mit der Adiponectinproduktion in Zusammenhang stehenden Krankheit oder Störung, umfassend:
(i) Pentosanpolysulfat (CAS 37300-21-3 N oder 116001-96-8, (C₅H₆Na₂O₁₀S₂)ₙ, n = 1-10), in Kombination mit
(ii) einer zweiten Verbindung, ausgewählt aus Indomethacin (CAS 53-86-1) und Pioglitazon (CAS 112529-15-4), wobei das Pentosanpolysulfat und die zweite Verbindung in einem molaren Verhältnis von 0,01:1 bis 1:0,01 bereitgestellt werden, vorzugsweise in einem Gewichtsverhältnis von 1:0,1 bis 0,9:1, weiter bevorzugt in einem Gewichtsverhältnis von 1:0,3 bis 1:1, wie etwa 1:0,5 bis 1:0,7,
wobei die Krankheit oder Störung eine neurodegenerative Störung ist.

2. Pharmazeutische Zusammensetzung zur Verwendung bei der Behandlung einer mit mitochondrialer Aktivität in Zusammenhang stehenden Krankheit oder Störung und/oder einer mit der Adiponectinproduktion in Zusammenhang stehenden Krankheit oder Störung nach Anspruch 1, wobei die Zusammensetzung ferner umfasst:
(iii) mindestens einen pharmazeutisch annehmbaren Träger.

## Revendications

1. Composition pharmaceutique pour une utilisation dans le traitement d'une maladie ou d'un trouble lié(e) à l'activité mitochondriale et/ou d'une maladie ou d'un trouble lié(e) à la production d'adiponectine, comprenant :
(i) du polysulfate de pentosane (CAS 37300-21-3 N ou 116001-96-8, (C₅H₆Na₂O₁₀S₂)ₙ, n=1-10), en combinaison avec
(ii) un second composé choisi parmi l'indométacine (CAS 53-86-1) et la pioglitazone (CAS 112529-15-4), dans laquelle le polysulfate de pentosane et le second composé sont fournis dans un rapport molaire de 0,01:1 à 1:0,01, de préférence dans un rapport pondéral de 1:0,1 à 0,9:1, de manière davantage préférée dans un rapport pondéral de 1:0,3 à 1:1, tel que 1:0,5 à 1:0,7,
dans laquelle la maladie ou le trouble est un trouble neurodégénératif.

2. Composition pharmaceutique pour une utilisation dans le traitement d'une maladie ou d'un trouble lié(e) à l'activité mitochondriale et/ou d'une maladie ou d'un trouble lié(e) à la production d'adiponectine selon la revendication 1, dans laquelle la composition comprend en outre
(iii) au moins un support pharmaceutiquement acceptable.
